(19) Europäisches Patentamt
European Patent Office
Office européen des brevets

(11) **EP 2 817 405 B1**

(12) **EUROPÄISCHE PATENTSCHRIFT**

(45) Veröffentlichungstag und Bekanntmachung des Hinweises auf die Patenterteilung:
**07.12.2016 Patentblatt 2016/49**

(51) Int Cl.:
*C12M 1/08* (2006.01)     *C12M 1/04* (2006.01)

(21) Anmeldenummer: **13706481.2**

(86) Internationale Anmeldenummer:
**PCT/EP2013/053393**

(22) Anmeldetag: **20.02.2013**

(87) Internationale Veröffentlichungsnummer:
**WO 2013/124329 (29.08.2013 Gazette 2013/35)**

(54) **EINWEG ABSCHEIDER ZUR RÜCKHALTUNG UND RÜCKFÜHRUNG VON ZELLEN**

ONE-WAY SEPARATOR FOR RETAINING AND RECIRCULATING CELLS

SÉPARATEUR À USAGE UNIQUE DESTINÉ À LA RÉTENTION ET AU RETOUR DE CELLULES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.02.2012 EP 12001121**

(43) Veröffentlichungstag der Anmeldung:
**31.12.2014 Patentblatt 2015/01**

(73) Patentinhaber:
• **Bayer Technology Services GmbH**
  **51368 Leverkusen (DE)**
• **Bayer Intellectual Property GmbH**
  **40789 Monheim (DE)**

(72) Erfinder:
• **PASTOR, Andre**
  **42719 Solingen (DE)**
• **SELETZKY, Juri**
  **94710-2428 Berkeley (US)**
• **BROD, Helmut**
  **51061 Köln (DE)**
• **KAULING, Joerg**
  **51427 Bergisch Gladbach (DE)**
• **COMMER, Peter**
  **53721 Siegburg (DE)**

(74) Vertreter: **BIP Patents**
  **c/o Bayer Intellectual Property GmbH**
  **Alfred-Nobel-Straße 10**
  **40789 Monheim am Rhein (DE)**

(56) Entgegenhaltungen:
| | |
|---|---|
| EP-A1- 0 471 947 | EP-A2- 0 599 651 |
| WO-A1-00/05337 | WO-A1-98/07828 |
| WO-A1-99/58222 | WO-A1-2009/139703 |
| WO-A1-2011/142670 | WO-A2-03/020919 |
| WO-A2-2009/152990 | DE-A1- 2 548 950 |
| DE-A1- 4 401 576 | DE-A1- 10 223 536 |
| DE-A1-102010 015 236 | DE-U- 7 215 337 |
| US-A- 4 814 278 | US-A- 5 350 527 |
| US-A- 5 698 102 | US-B1- 6 959 618 |

**Beschreibung**

Hierin wird ein Feststoffschrägkanalabscheider mit Lamellenpaket zur Rückhaltung von Feststoffen aus einem Reaktorgemisch nach Anspruch 1 beschrieben.

Die Züchtung tierischer und pflanzlicher Zellen hat große Bedeutung bei der Herstellung biologisch aktiver Substanzen und pharmazeutisch aktiver Erzeugnisse. Insbesondere die Züchtung von Zellen, welche häufig in einem Nährmedium in freier Suspension durchgeführt wird, ist anspruchsvoll, weil die Zellen im Gegensatz zu Mikroorganismen sehr empfindlich hinsichtlich mechanischer Scherbeanspruchung und unzureichender Versorgung mit Nährstoffen sind.

Meist werden tierische und pflanzliche Zelllinien absatzweise gezüchtet. Dies hat den Nachteil, dass eine optimale Versorgung der Zellen infolge der sich ständig verändernden Substrat-, Produkt- und Biomassekonzentrationen nur schwer zu erreichen ist. Am Ende der Fermentation reichern sich außerdem Nebenprodukte an, z.B. Bestandteile abgestorbener Zellen, die meist unter großem Aufwand bei der späteren Aufarbeitung entfernt werden müssen. Aus den genannten Gründen, insbesondere aber bei der Herstellung instabiler Produkte, die z.B. durch proteolytische Angriffe beschädigt werden können, werden daher kontinuierlich betriebene Bioreaktoren verwendet.

Mit kontinuierlichen Bioreaktoren lassen sich hohe Zelldichten und eine damit verbundene hohe Produktivität erreichen, wenn folgende Anforderungen erfüllt sind:

- eine ausreichende und scherarme Versorgung der Zellen mit Substraten, insbesondere dem gelöstem Sauerstoff,

- eine ausreichende Entsorgung des bei der Veratmung entstehenden Kohlendioxids,

- ein effektives, scherarmes, verstopfungssicheres Zellrückhaltesystem zum Aufbau hoher Zellkonzentrationen,

- die Langzeitstabilität (Sterilität, Hydrodynamik) des Bioreaktors und Rückhaltesystems.

Neben der kontinuierlichen Fahrweise kann ein Bioreaktor mit einem effizienten Zellrückhaltesystem z.B. auch zur Anzucht von Vorkulturen mit besonders hohen Zelldichten verwendet werden. Man verwendet dann das Zellrückhaltesystem auf diskontinuierliche Weise im Repeated-Batch-Modus, um Zellkulturüberstand nahezu frei von Biomasse abzuziehen. Danach kann der Vorkulturreaktor wieder mit frischem Nährmedium aufgefüllt werden, um die Kultur auf diese Weise zu höheren Zelldichten zu bringen als bei einfachem absatzweisen Betrieb.

Damit eine hohe Zelldichte (> 20 Millionen lebende Zellen pro Milliliter) in einem kontinuierlich betriebenen Bioreaktor erreicht werden kann, ist eine effiziente Rückhaltung der Zellen notwendig. Der erforderliche Rückhaltegrad hängt dabei von der Wachstumsrate der Zellen und der Perfusionsrate $q/V$ (Mediendurchsatz $q$ pro Bioreaktorvolumen $V$) ab.

In der Vergangenheit wurden unterschiedliche Zellrückhaltesysteme für kontinuierlich betriebene Bioreaktoren vorgeschlagen, welche zumeist außerhalb des Bioreaktors angeordnet werden. Grund hierfür ist die leichte Zugänglichkeit des Zellrückhaltesystems zu Wartungs- und Reinigungszwecken.

Um Schädigungen der Zellen insbesondere aufgrund unzureichender Sauerstoffversorgung und Kohlendioxidentsorgung außerhalb des Bioreaktors so klein wie möglich zu halten, sind Zellrückhaltesysteme mit kleinen Arbeitsvolumina und damit verbundenen kurzen Verweilzeiten der Zellen wünschenswert.

Neben Membranfiltern, Apparaten, die nach dem Prinzip der Querstromfiltration mit feststehenden und beweglichen Membranen arbeiten, kommen im Stand der Technik spezielle Zentrifugen und Schwerkraftabscheider zum Einsatz.

Im Fall der Zellrückhaltung mit Hilfe von Membranfiltern werden Ablagerungen bzw. Verschmutzungen beobachtet, die einen zuverlässigen und wartungsfreien Langzeitbetrieb verhindern können. Die Ablagerungen können reduziert werden, wenn die Membranflächen hinreichend schnell überströmt werden. Dies kann in stationärem oder oszillierendem Betrieb erreicht werden. Ein Beispiel für ein oszillierend überströmtes Membransystem ist das Alternating Tangential Flow (ATF) System der Firma Refine Technologies Inc.). Die schnelle Überströmung der Membranflächen läuft jedoch der Grundvoraussetzung an eine scherarme Zellkultivierung zuwider.

Scherarme Zentrifugen zur Abscheidung von Zellen im Zentrifugalfeld arbeiten nur über wenige Wochen ohne Wartungsaufwand und machen einen Austausch der Zentrifugenelementen erforderlich. Hiermit vergrößert sich das Risiko von Kontaminationen.

Die bei der Züchtung von Zellen überwiegend verwendeten Schwerkraftabscheider sind Absetzbehälter und Schrägkanalabscheider. Verglichen mit einfachen Absetzbehältern haben die Schrägkanalabscheider in großen Maßstäben den Vorteil eines beträchtlich geringeren Volumens in Verhältnis zur Abscheidefläche. Eine Veröffentlichung (Henzler, H.-J., Chemie-Technik, 1, 1992, 3) beschreibt die Zellrückhaltung in Schrägkanalabscheidern, welche im Gegenstrom, Kreuzstrom und Gleichstrom betrieben werden können. Der durchströmte Kanalquerschnitt kann mit Platten oder Rohren versehen sein. WO1994026384 A1 beansprucht den Einsatz von Schrägkanalabscheidern zur Rückhaltung von Zellen in Gegenstromabscheidem. In WO2003020919 A2 werden unter anderem Gegen- und Kreuzstromabscheider, sowie Kombinationen mit verschiedenen Vorabscheidem (z.B. Hydrozyklonen) für die Rückhaltung von Zellen beschrieben. Diese bekannten Schrägkanalabscheider werden aus Edelstahl hergestellt, und deren Elemente geschnitten, aufwändig geschliffen, spiegelpoliert und zusammengeschweißt.

Die Schrägkanalabscheider werden über einen externen Kreislauf an den Bioreaktor angeschlossen. Hierzu sind Schlauchleitungen und Pumpen erforderlich.

Um die Stoffwechselaktivität und das Anbacken von Zellen in einem Schwerkraftabscheider zu vermindern, wird das Herunterkühlen der Zellkultur**brühe** auf **ihrem** Weg zum Schwerkraftabscheider vorgeschlagen. Eine reduzierte Stoffwechselaktivität bei niedriger Temperatur ist bei längerem Aufenthalt der Zellen außerhalb des Bioreaktors sicherlich von Vorteil.

WO2009152990(A2) beschreibt ein Zellrückhaltesystem zur Rückhaltung und Rückführung von Zellen in einem durchströmten Gefäß umfassend eine Vielzahl von nebeneinander angeordneten Kanälen, wobei die Kanäle einen stehenden Hohlzylinder bilden und gegenüber der Längsachse des Hohlzylinders um einen Winkel $\beta$ zwischen 10° und 60°, geneigt sind. Das durchströmte Gefäß kann ein Bioreaktor oder ein mit einem Bioreaktor verbundenes Gefäß zur Zellrückhaltung und - rückführung sein. Die Kanäle sind am unteren Ende geöffnet. Am oberen Ende führen sie in einen gemeinsamen Ringraum, der über mindestens eine Leitung verfügt, über die ein Erntestrom aus dem Gefäß befördert werden kann. In den Kanälen erfolgt die Separation von Zellen und Zellkulturlösung. Durch die kontinuierliche Entnahme des Erntestroms aus dem Bioreaktor werden Zellkulturlösung und Zellen in die Kanäle eingesaugt. Die Zellen sedimentieren innerhalb der schräg angeordneten Kanäle und rutschen wie in klassischen Schrägkanalabscheidern im Gegenstrom zum zufließenden Erntestrom wieder aus den Kanälen heraus und verbleiben damit im Gefäß. Die von den Zellen getrennte Zellkulturlösung wird durch die Kanäle in den Ringraum oberhalb der Kanäle und schließlich aus dem Gefäß befördert.

Bei der stark regulierten pharmazeutischen Produktion entfällt ein großer zeitlicher, technischer und personeller Aufwand auf die Bereitstellung gereinigter und sterilisierter Bioreaktoren und Bioreaktorelemente wie z. B. Zellrückhaltesysteme. Um Kreuzkontaminationen bei einem Produktwechsel in einer Multi-Purpose-Anlage oder zwischen zwei Produktchargen sicher zu vermeiden, wird außer der Reinigung eine sehr aufwendige Reinigungsvalidierung benötigt, welche bei einer Prozessadaption ggf. wiederholt werden muss. Zur Reinigung und Sterilisation konventioneller Batch, Fed-Batch oder Perfusionsfermenter aus Edelstahl kommt in der Regel die Clean-in-Place (CIP)-Technik in Kombination mit der Steam-in-Place ( SIP )-Technik in sog. fest verrohrten Anlagen zum Einsatz. Um bei kontinuierlicher Prozessführung eine ausreichende Langzeitsterilität zu gewährleisten, wird auch die Autoklavier-Technik genutzt, die allerdings einen umständlichen Transport der Reaktoren oder Reaktorelemente zum Autoklaven erfordert und nur in vergleichsweise kleinen Reaktormaßstäben anwendbar ist. Die Gefahr der Kontamination ist besonders kritisch bei Verwendung von alternden Verschleißteilen, z.B. gedichteten Rührerwellen, unsachgemäßer Sterilisation oder Anlagentransport, der Inbetriebnahme bzw. dem Verbinden von Anschlussleitungen nach Autoklavieren und der regelmäßigen Probeentnahme.

Bei im Batch- oder Fed-Batch-Modus genutzten CIP/SIP-Anlagen kann der durch die Bereitstellungsprozeduren bedingte Nutzungsausfall eines Reaktors insbesondere bei häufigem Produktwechsel infolge der kurzen Nutzungsperioden in der Größenordnung der Reaktorverfügbarkeit liegen.

Um der Forderung an ein schnelles und flexibles Neubeschicken der Produktionsanlage unter Wahrung maximaler Sauberkeit und Sterilität gerecht zu werden, erfreuen sich auf dem Markt Konzepte für Einweg-Reaktoren eines ständig wachsenden Interesses.

Es stellt sich damit ausgehend vom Stand der Technik die Aufgabe, eine effiziente Methode zur Rückhaltung und Rückführung von tierischen, insbesondere humanen, und pflanzlichen Zellen in einem kontinuierlich oder absatzweise betriebenen Verfahren bereitzustellen, die der Empfindlichkeit der Zellen hinsichtlich mechanischer Scherbeanspruchung und der ausreichenden Versorgung der Zellen mit Nährstoffen Rechnung trägt, die bis in sehr große Maßstäbe skalierbar ist, die den wartungs-, reinigungs- und sterilitätstechnischen Anforderungen der pharmazeutischen Industrie gerecht wird, deren Verwendung die Komplexität und das Fehlerrisiko erniedrigt, und die bei minimalem Ressourceneinsatz eine ökonomisch und ökologisch optimale Nutzung (Herstellung und Entsorgung) als Einwegsysteme ermöglicht.

Die oben genannte Aufgabe wurde durch einen Feststoffschrägkanalabscheider mit Lamellenpaket zur Rückhaltung von Feststoffen aus einem Reaktorgemisch gemäß Anspruch 1 gelöst.

Der erfindungsgemäße Feststoffschrägkanalabscheider umfasst zur Rückhaltung von insb. Zellen aus einem Bioreaktorgemisch folgenden Elemente:

- einen oberen Bereich des Feststoffabscheiders mit einer oder mehreren Durchführungen/Einbauten (80) zum Abziehen eines von den Zellen getrennten Erntestromes (70) (=Harvest) aus einem Erntestromsammelbereich 56, verbunden mit
- einem Abscheidebereich gebildet durch ein Lamellenpaket (1) aus ein oder mehrlagigen Kunststoffstegplatten, das während des Betriebs mit einem Winkel (10) von 30° bis 80° zur Horizontalen geneigt ist, verbunden mit

- einem unteren Segment des Feststoffabscheiders mit einer oder mehreren Durchführungen oder Einbauten (84) zur Strömungsverteilung des Reaktorgemisches (74), über - einen sich nach unten konisch oder pyramidal verjüngten Feststoffsammelbereich (57) zum Sammeln der Zellen mit Hilfe der Schwerkraft.

Bevorzugt weist der nach unten verjüngte, insb. konisch oder pyramidal, Feststoffsammelbereich (57) einen Winkel (58, 59) von 10° bis 60° gegen die Vertikale auf. Die Winkel 58 und 59 können separat ausgewählt werden.

Um die Rückführung zu ermöglichen weist der Feststoffsammelbereich (57) eine oder mehrere Durchführungen (89) oder ggf. Einbauten (88) zum Abziehen der Zellen auf. Beispiel für Einbauten ist eine zentrale Absaugung.

Die ein oder mehrlagigen Kunststoffstegplatten bilden Kanäle und das Lamellenpaket 1 besteht bevorzugt aus einer Vielzahl von nebeneinander angeordneten Kanälen.

Die Kanäle sind am unteren Ende und am oberen Ende geöffnet. Am unteren Ende führen die Kanäle zu dem gemeinsamen unten konisch verjüngten Feststoffsammelbereich 57. Am oberen Ende führen sie zu einem gemeinsamen Erntestromsammelbereich 56, der über mindestens eine Durchführung 80 verfügt, durch die der Erntestrom aus dem Gefäß befördert werden kann.

In den Kanälen der erfindungsgemäßen Feststoffschrägkanalabscheider erfolgt die Separation von Zellen und Zellkulturlösung. Durch die kontinuierliche Entnahme des Erntestroms aus dem Bioreaktor werden Zellkulturlösung und Zellen in die Kanäle eingesaugt. Die Zellen sedimentieren innerhalb der schräg angeordneten Kanäle und rutschen wie in klassischen Schrägkanalabscheidern im Gegenstrom zum zufließenden Erntestrom wieder aus den Kanälen heraus und sammeln sich in dem konisch verjüngten Feststoffsammelbereich 57. Üblicherweise verfügt der Feststoffsammelbereich 57 über einen oder mehrere Durchführungen/Einbauten 88/89, verbunden mit dem Bioreaktor zur Absaugung der eingesammelten Zellen und Rückführung in den Bioreaktor.

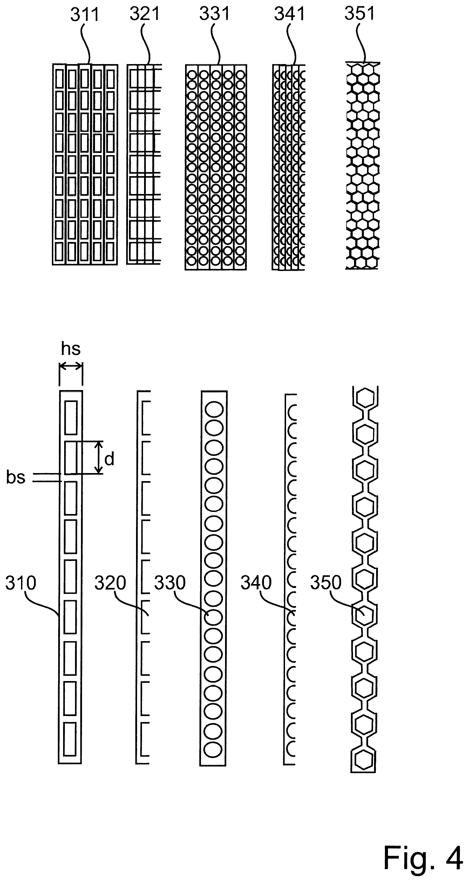

Die Kanäle des Lamellenpakets 1 können einen eckigen, elliptischen, runden oder halbrunden Querschnitt haben (Fig 4).

Die Dimensionierung der Kanäle (Anzahl, Durchmesser, Länge) hängt jeweils von der Art der zurückzuhaltenden Zellen, der Größe des Bioreaktors und dem Durchsatz ab.

Die Kanalbreite $d$ liegt bevorzugt bei $d \geq 3$ mm, um eine Verstopfung der Kanäle zu verhindern. In einer bevorzugten Ausführungsform werden Kanäle mit einer Kanalbreite von 3 mm bis 100 mm, bevorzugt von 4 mm bis 20 mm, besonders bevorzugt von 3 -7 mm eingesetzt, um einerseits Verblockungszustände sicher zu vermeiden, andererseits aber das die Raum-Zeit-Ausbeute mindernde Volumenverhältnis von Abscheider- und Bioreaktorraum möglichst gering zu halten.

Die erforderliche Abscheidefläche $A_{erf}$ ergibt sich aus der Sedimentationsgeschwindigkeit $ws$, der Perfusionsrate $q/V$ (Mediendurchsatz $q$ pro Bioreaktorvolumen $V$) und dem Bioreaktorvolumen nach Gl. 1. Ein Wirkungsgrad $\eta$ berücksichtigt die Leistungsminderung von Schrägkanalabscheidern gegenüber Vertikalabscheidern (Gl. 2).

Die theoretische Abscheidefläche $A_{th}$ bei rechteckigen und zylindrischen Querschnitten kann nach in der Literatur (H.-J. Binder, Sedimentation aus Ein- und Mehrkornsuspensionen in schräg stehenden, laminar durchströmten Kreis- und Rechteckrohren, Dissertation Berlin, 1980) veröffentlichen Ansätzen aus den Gln. 3 und 4 näherungsweise bestimmt werden:

$$A_{erf} = \frac{Perfusionsrate \cdot V}{ws} \qquad (\text{Gl. 1})$$

$$A_{th} = \frac{A_{erf}}{\eta} \qquad (\text{Gl. 2})$$

$$\text{Rechteck:} \quad A_{th} \approx Z \cdot \sin(\beta) \cdot d \cdot L \qquad (\text{Gl. 3})$$

$$\text{Zylinder:} \quad A_{th} \approx \frac{3 \cdot \pi}{16} \cdot Z \cdot \sin\beta) \cdot d \cdot L \qquad (\text{Gl. 4})$$

Hierbei sind Z die Anzahl der Kanäle, $\beta$ der Winkel, um den die Kanäle gegenüber der Richtung der Schwerkraft gekippt sind, $d$ der Innendurchmesser und $L$ die Länge der Kanälen. $\pi$ ist die Kreiszahl ($\pi$ = 3,14159...).

Bei der Dimensionierung der Kanallänge ist die Einhaltung laminarer Strömungsbedingungen (Reynolds-Zahl Re < 2300) zu berücksichtigen.

Der dynamische Druck an der Erntestromabzugsstelle (=Durchführuugen/Einbauten 80) sollte dabei mindestens um das 5- bis 10- fache kleiner sein als der Druckverlust in den Kanälen, um das wirkungsgradmindernde Phänomen der Maldistribution auszuschließen. Ausreichende Druckverluste sind bei Kanallängen ab 0,1 m als technisch realisierbar anzusehen, während bevorzugt Kanallängen von 0,2 m bis 5 m, besonders bevorzugt Kanallängen von 0,4 m bis 2 m realisiert werden.

Kurze Kanallängen L können aufgrund der reduzierten Druckverluste zu Verteilungsproblemen führen, was insbesondere bei der Abnahme des Erntestroms **aus** dem oberen Erntestromsammelbereich 56 eine **Verteilungsvorrichtung** zur Reduzierung der Abzugsgeschwindigkeiten erfordern kann. Optional weisen daher die Durchführungen/Einbauten 80 Strömungsinverter 81 für ein vergleichmäßigtes Abziehen des von den Zellen getrennten Erntestroms 70 (=Harvest) aus einem Erntestromsammelbereich 56 auf.

Der erfindungsgemäße Feststoffschrägkanalabscheider kann üblicherweise 1 bis $10^6$ Kanäle umfassen, bevorzugt 10 bis 100.000, besonders bevorzugt 10 bis 10.000. Die Kanäle sind ggf. über eine oder mehrere Stegplatten in einem Lamellenpaket 1 zur Optimierung des Platzbedarfs verteilt. Bevorzugt umfasst das Lamellenpaket 1 von 1 bis 400 Stegplatten, besonders bevorzugt 1 bis 50 Stegplatten maßstababhängig.

Das Breiten- zu Höhenverhältnis des Lamellenpaketes 1 bestehend aus ein- oder mehrlagigen Stegplatten einschließlich der Stützplatte lässt sich anpassen. Lamellenpakete 1 mit quadratischem, zylindrischem rechteckigem oder elliptischem Querschnitt mit einem Höhen- zu - Breiten-Verhältnis H/D von $0{,}005 \leq H/D \leq 1{,}5$, bevorzugt $0{,}02 \leq H/D \leq 1{,}2$, besonders bevorzugt $0{,}1 \leq H/D \leq 1{,}0$ werden vorzugsweise eingesetzt.

Vorzugsweise weist der Abscheidebereich mehrere aufeinander gestapelte Kunststoffstegplatten auf, die einen Grundkörper bilden.

Alternativ kann das Lammellenpaket 1 aus einer profilierten Platte 340 oder 320 gebildet werden (siehe Figur 4). Eine profilierte Platte weist bevorzugt eine glatte Seite und eine Seite mit einer Folge von Stegen und Rinnen in gleichbleibenden Abständen auf. Kanäle bilden sich bei der Stapelung der Platte in einer oder mehreren Lagen z.B. auf eine Stützplatte 30. Dabei werden die Rinnen zur offenen Seite hin jeweils durch die glatte Seite einer benachbarten Lage bzw. durch die Wand des Stators verschlossen. Es besteht ebenfalls die Möglichkeit ein Lamellenpaket oder -teilpaket ein- oder mehrlagig zu extrudieren und zu einem Lamellenpaket 1 zu verbinden.

Eine bevorzugte Verbindung der Stegplatten erfolgt über Verklebung oder Verschweißung Das Lamellenpaket sollte durch die Verbindung in erster Linie räumlich fixiert werden. Außerdem wird angestrebt, die sog. Totzonen (nicht zur Abscheidung genutzte Räume um die Außenflächen der Stegplatten) möglichst klein zu halten. Eine vollständige Vermeidung dieser Totzonen ist dabei jedoch nicht unbedingt gefordert. Als Kleber eignen sich die dem Fachmann bekannten, auf die Material- und Oberflächeneigenschaften der Kanäle abgestimmten Klebekomponenten. Insbesondere wird bevorzugt Kleber verwendet, der in den geforderten FDA-Güteklassen im Markt zur Verfügung steht. Für das Verschweißen lassen sich thermische Verbindungstechniken wie Hitze, Laser, Ultraschall anwenden. Eine besonders bevorzugte Verbindungstechnik ist das Laserstrahlschweißen, das insbesondere auch in Kombination mit dem Zuschnitt des Lamellenpaketes in einer dazu geeigneten Vorrichtung angewendet werden kann. Die Schweißtechnik besitzt den Vorteil, dass die Anzahl der in den Pharmaprozess eingeführten Kunststoffe durch diese Verbindungstechnik nicht erhöht wird.

Die Geometrie der Kanäle wird durch das Verhältnis der Steghöhe hs, zur Kanalbreite d festgelegt. Technisch realisierbare hs/ d -Verhältnisse liegen je nach Beschaffenheit (Formbarkeit, Elastizität, Tiefziehvermögen) im Bereich $0{,}01 \leq hs/d \leq 5$. Hierbei ist zu beachten, dass beide Abmessungen hs und $d$ jeweils größer als oder gleich 3 mm, bzw. bevorzugt größer als oder gleich 5 mm sein sollten. Bevorzugte hs/ $d$ -Verhältnisse liegen bei 0,5 bis 5. Die Stegbreiten bs werden durch die mechanische Stabilität des Folienmaterials bestimmt. Die Stegbreiten bs sollten möglichst klein sein, um hohe Abscheideflächen pro Abscheidervolumen zu ermöglichen. Gleichzeitig sollten sie nicht zu gering gewählt werden, um eine kraftschlüssige Verbindung mit der unteren Lage ohne Formveränderung erlauben zu können. Bei extrudierten Lamellenpaketen 1, oder bei Lamellenpaketen, die aus extrudierten Lamellenteilpaketen oder Stegplatten aufgebaut sind, können sehr hohe Steifigkeiten mit kleinen Stegbreiten ohne großen Verlust an Abscheidefläche realisiert werden, so dass diese Herstellungsform bevorzugt ist.

Die profilierte Platte kann durch Formgebung unmittelbar bei der Plattenherstellung oder durch (z.B. klebetechnische) Verbindung einer geprägten, heiß oder kalt verformten Platte mit einer glatten Platte erfolgen. Die Materialeigenschaften der geprägten und glatten Platte können auf ihre unterschiedliche Funktionalität (gute Gleiteigenschaften und Formstabilität der geprägten Platte, gute Dichteigenschaften der glatten Platte) hin optimal, d.h. durch Wahl eines geeigneten, dem Fachmann bekannten Werkstoffes mit entsprechender Oberflächengüte, angepasst werden.

Üblicherweise werden kommerziell verfügbare, kostengünstige, und für Pharma-Prozesse geeignete Kunststoffstegplatten z.B. aus Polykarbonat als Lamellenteilpakete zur Herstellung des Lamellenpakets 1 in der passenden Länge geschnitten bzw. hergestellt und aufeinander befestigt. Diese Stegplatten sind als Meterware mit fertiger Kanalgeometrie (Sedimentationsfläche) und gebrauchsfertiger Oberflächenqualität extrudiert. Das Zuschneiden insbesondere Ablängen auf das benötigte Längenmaß erfolgt üblicherweise durch Absägen z. B. auf einer Kreissäge. Üblicherweise dienen die Längsstege einerseits als Gehäuse des Abscheiders und andererseits der Stabilisierung der Strömungskanäle, und die Querstege dienen einerseits als Gehäuse des Abscheiders und bilden andererseits die Abscheiderfläche.

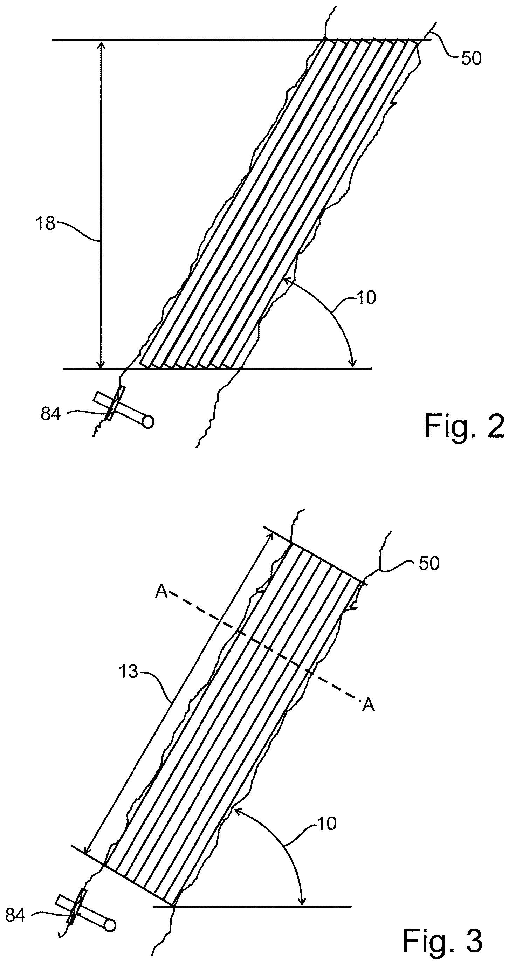

Das aus Kunststoffstegplatten aufgebaute Lamellenpaket ist entweder als gerader Quader (Fig.3) ausgeführt, wobei die Ebene der Kanalöffnungen rechtwinklig zur Auflagefläche des Lamellenpaketes 1 ist, oder als schiefer Quader (Fig. 2), wobei die Kanalöffnungen im eingebauten Zustand auf einer horizontalen Ebene liegen. Letztere Lösung ist bevorzugt, um einen durch Sedimentation verursachten Konzentrationsgradienten zu den unteren Kanalöffnungen zu verhindern. Die Kanäle werden vom Reaktorgemisch vergleichmäßigt angeströmt, ggf. mit Hilfe von horizontalen Verteiler 85.

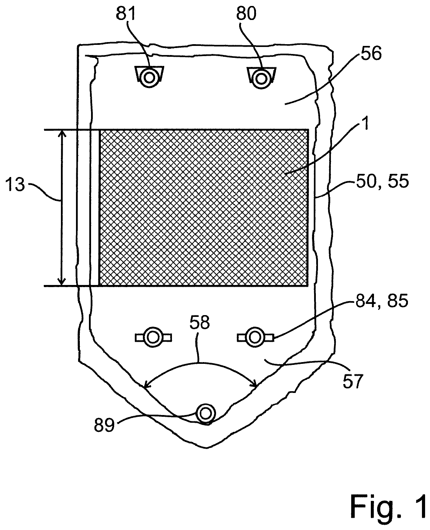

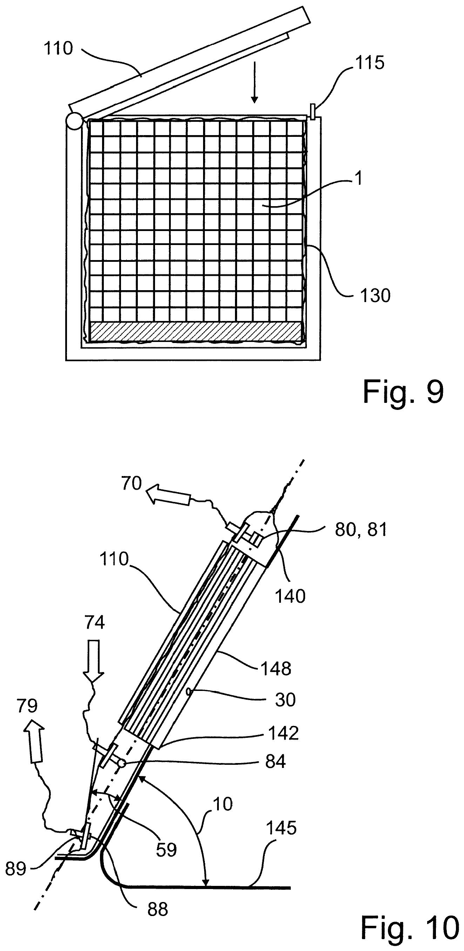

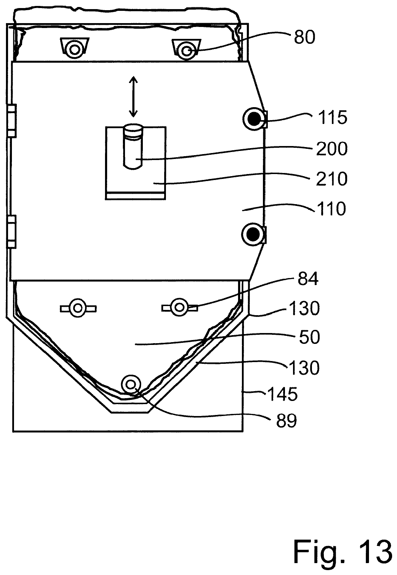

**In einer ersten Ausführungsform** (Fig. 1 bis 13) sind die Elemente des erfindungsgemäßen Einweg-Feststoffschrägkanalabscheiders in einem durchströmbaren, gamma-sterilisierbaren Kunststoffbeutel eingebaut. Im oberen Segment des mittleren Bereichs des Kunststoffbeutels ist das Lamellenpaket 1 aus ein oder mehrlagigen Kunststoffstegplatten eingebracht. Der Kunststoffbeutel begrenzt außerdem den Erntestromsammelbereich 56 und den konisch verjüngten Feststoffsammelbereich, wobei der Feststoffsammelbereich 57 bevorzugt einen Winkel 58, 59 von 10° bis 60° gegen die Vertikale aufweist. Im unteren Segment des mittleren Bereiches des Kunststoffbeutels weisen Durchführungen oder Einbauten 84 einen horizontalen Verteiler 85 zur gleichmäßigen horizontalen Strömungsverteilung der Zellkulturlösung (=Feed) 74 über eine Einleitfläche 510.

Die oben genannte Aufgabe wird in dieser Ausführungsform durch einen Einweg-Feststoffschrägkanalabscheider zur Rückhaltung und Rückführung von Zellen aus einem Bioreaktorgemisch gelöst, umfassend einen durchströmbaren, gamma-sterilisierbaren Kunststoffbeutel mit folgenden Einbauten:

- Im oberen Bereich des Kunststoffbeutels ein oder mehreren Durchführungen/Einbauten 80 zum Abziehen eines von den Zellen getrennten Erntestromes 70 (=Harvest) aus einem Erntestromsammelbereich 56
- im oberen Segment eines mittleren Bereiches des Kunststoffbeutels einen Abscheidebereich gebildet durch ein Lamellenpaket 1 aus ein oder mehrlagigen Kunststoffstegplatten, das während des Betriebs mit einem Winkel (10 = $\beta$) von 30° bis 80° zur Horizontalen geneigt ist,
- im unteren Segment des mittleren Bereiches des Kunststoffbeutels eine oder mehrere Durchführungen oder Einbauten 84 zur Strömungsverteilung des Reaktorgemisches (74) optional mit horizontalen Verteiler 85 zur gleichmäßigen horizontalen Strömungsverteilung der Zellkulturlösung (=Feed) 74 über eine Einleitfläche 510,
- Im unteren Bereich des Kunststoffbeutels einen unten konisch oder pyramidal verjüngten Feststoffsammelbereich 57 zum Sammeln der Zellen mit Hilfe der Schwerkraft. Üblicherweise weist der Feststoffsammelbereich 57 eine oder mehrere Durchführungen 89 oder Einbauten 88 zum Abziehen der Zellen auf.

Der obere Bereich des Kunststoffbeutels kann auch nach oben verjüngt sein.

Üblicherweise wird der Kunststoffbeutel aus einem ein- oder mehrlagigen durchsichtigen Polymermaterial ausgeführt, dass einen Einblick in die Vorrichtung während des Betriebs ermöglicht. Das Polymermaterial gestattet bei üblichen geringen Filmdicken von s « 1 mm Apparate mit einem vergleichsweise kleinem Masseanteil. Es ist kostengünstig zu beschaffen und zu verarbeiten, was sich sehr gut für den Aufbau von Einwegsystemen eignet. Die Entsorgung der gebrauchten Abscheider und die Verwendung eines neuen Einwegabscheiders sind damit wirtschaftlicher als die Reinigung von gebrauchten Abscheidevorrichtungen, insbesondere da bei der Verwendung von Einweg-Abscheidern eine teure Reinigung mit Wasser für Injektionen (Water for Injections, WFI) und die zeitaufwändige Reinigungsvalidierung entfällt. Der erfindungsgemäße Abscheider ist vorzugsweise steril verpackt.

Als Materialien für den Kunststoffbeutel eignen sich insbesondere die in der Patentschrift US 6,186,932 B1 in den Spalten 2 und 3 für die dort genannten Transportbeutel (*sachets*) verwendeten Materialien und Materialkombinationen. Auch die dort aufgeführten Wandstärken lassen sich auf die erfindungsgemäße Abscheidevorrichtung übertragen.

In einer bevorzugten Ausführungsform bestehen die Wände des Kunststoffbeutels aus einem dem Fachmann bekannten Folienverbundmaterial aus zwei oder mehreren Schichten (Laminat oder Co-Extrudat), um die Eigenschaften des Kunststoffbeutels hinsichtlich Entfaltungsverhalten, Dehnungsverhalten, Gasdiffusion, Stabilität, Prozessverträglichkeit (minimale Adsorption von Produkten und Zellen) und Schweißbarkeit zu verbessern.

Bei der Dimensionierung der Kanallänge ist die Einhaltung laminarer Strömungsbedingungen (Reynolds-Zahl Re < 2300) zu berücksichtigen. Die Kanallänge L richtet sich nach der Länge der zur Verfügung stehenden Beutelinnenabmessung (= Länge des Beutels LK). Die zu realisierende Länge LK des Beutels richtet sich nach dem in dem Kunststoffbeutel zu realisierenden Füllständen und nach den im Kunststoffbeutel zu realisierenden hydrostatischen Drücken. Zu große hydrostatische Drücke können ggf. an entsprechend dimensionierte, nicht produktberührte und daher wiederverwendbare Einhausungen weitergegeben werden.

Üblicherweise beträgt die Kanallängen L 30 % bis 95%, besonders bevorzugt 60% bis 90% der Länge LK des Kunststoffbeutels.

Der erfindungsgemäße Feststoffabscheider mit Kunststoffbeutel aus Polymerfolien kann beispielsweise nach dem in US 6,186,932 B1 beschriebenen Verfahren hergestellt werden, wobei die Schweißnähte angepasst werden müssen. Ausführungsbeispiele für die Herstellung von bevorzugten Ausführungsformen der erfindungsgemäßen Abscheidevorrichtung sind weiter unten beschrieben.

Durchführungen werden üblicherweise aus demselben Material hergestellt, aus dem auch die produktberührte Folie besteht, um mit dieser eine steril- und festigkeitstechnisch einwandfreie Verschweißung zu ermöglichen. Bevorzugtes produktberührtes Filmmaterial ist Polyethylen verschiedener dem Fachmann bekannter Vernetzungsgrade. Als Außenmantelfolien kommen je nach Anwendung und Prozessanforderung verschiedene dem Fachmann bekannte Materialien mit einem gegenüber der Innenfolie vergrößerten Schmelzpunkt für den Einsatz von thermischen Schweißverfahren und/oder besseren Festigkeits- und/oder Diffusionseigenschaften zum Einsatz.

Üblicherweise werden die Stegplatten an eine Stützplatte 30 gebunden, die Halt bietet und zur exakten Positionierung mit dem Kunststoffbeutel durch Kleben oder Schweißen verbunden werden kann.

In 3D-Beutel (aus 4 Folienbahnen zusammengeschweißte Beutel) lassen sich günstiger Weise Lamellenpakete 1 mit quadratischem, zylindrischem rechteckigem oder elliptischem Querschnitt mit einem Höhen- zu - Breiten-Verhältnis H/D von 0,3 < H/D < 1,5, bevorzugt 0,6< H/D < 1,2, besonders bevorzugt 0,9 < H/D < 1,0 einsetzen.

Für die einfacheren, preiswerteren 2D-Beutel ( aus zwei Folienbahnen zusammengeschweißte Beutel ) eignen flache Lamellenpakete mit rechteckigem Querschnitt sich H/D-Verhältnisse von 0,005 < H/D < 1, bevorzugt 0,02 < H/D < 0,6,besonders bevorzugt 0,1 < H/D < 0,4. Je nach Höhe des Lamellenpakets kann es für die Anfertigung eines 2D-Beutels einen bestimmten Abstand zwischen das Lamellenpakets und der Anfang der Verjüngung(en) übrig zu lassen.

Zur Herstellung des Abscheiders werden außerdem in einer Kunststofffolie die Durchführungen und weitere Einbauten an den passenden Stellen vorbereitet und ggf. eingebaut.

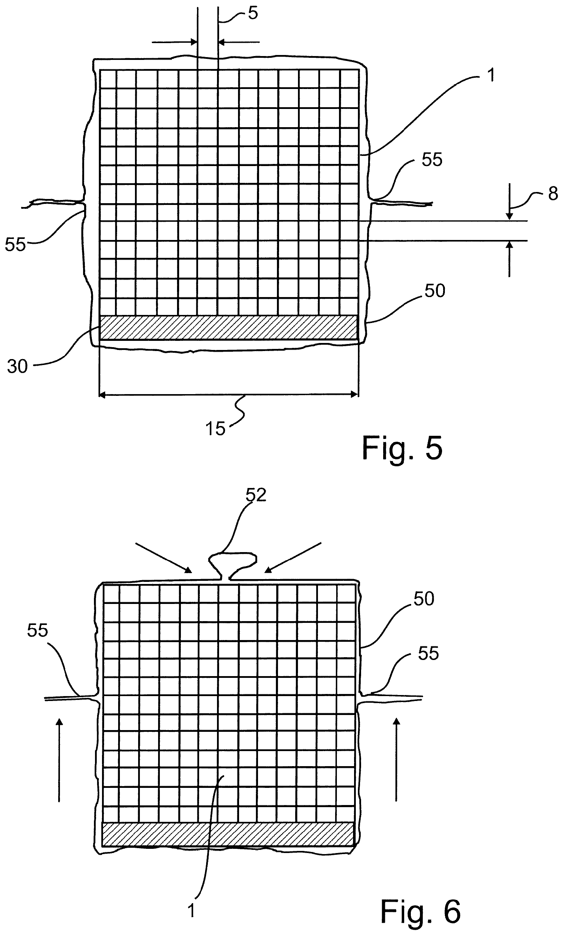

Anschließend wird ein Kunststoffbeutel 50 aus der Kunststofffolie, die das Lamellenpaket 1 einschließt, in einem Kunststoffbeutel 50 mit Schweißnaht 55 zusammengeschweißt (Fig. 5).

Das Lamellenpaket 1 einschließlich Stützplatte wird dann üblicherweise gegen die Innenflächen des Kunststoffbeutels 50 verpresst, um das Eindringen von Zellen zwischen Kunststoffbeutel 50 und Lamellenpaket 1 und damit Fouling zu verhindern.

In einer ersten Ausführungsform des Herstellungsverfahrens wird der Kunststoffbeutel 50 an das Lamellenpaket 1 gestrafft (Fig. 5) und die gebildete Falte 52 wird mittels eines oder mehrerer Befestigungsbänder 60 flach gedrückt und befestigt (Fig. 6). Als Befestigungsband eignet sich auch eine Kunststofffolie, die um Beutel und Lammellenpaket straff herumgewickelt wird. Günstige Straffungseigenschaften besitzen z.B. Haushaltsfolien ober flexible, dünne Folien aus Silikon. Auch eine Verschweißung des Lamellenpaketes 1 mit der Beutelwand kann zur Herstellung einer dichten Verbindung zwischen Beutel und Lamellenpaket geeignet sein.

Für den Betrieb wird die erfindungsgemäße Vorrichtung mit einem Winkel 10 = $\beta$ zur Horizontale gerichtet. Der Winkel $\beta$ richtet sich nach dem Absetz- und Abrutschverhalten der Zellen/Feststoffe und beträgt während des Betriebs $30° \leq \beta \leq 80°$ zur Horizontalen. In einer bevorzugten Ausführungsform liegt der Winkel $\beta$ von 35° bis 75°, besonders bevorzugt von 45° bis 60° zur Horizontalen.

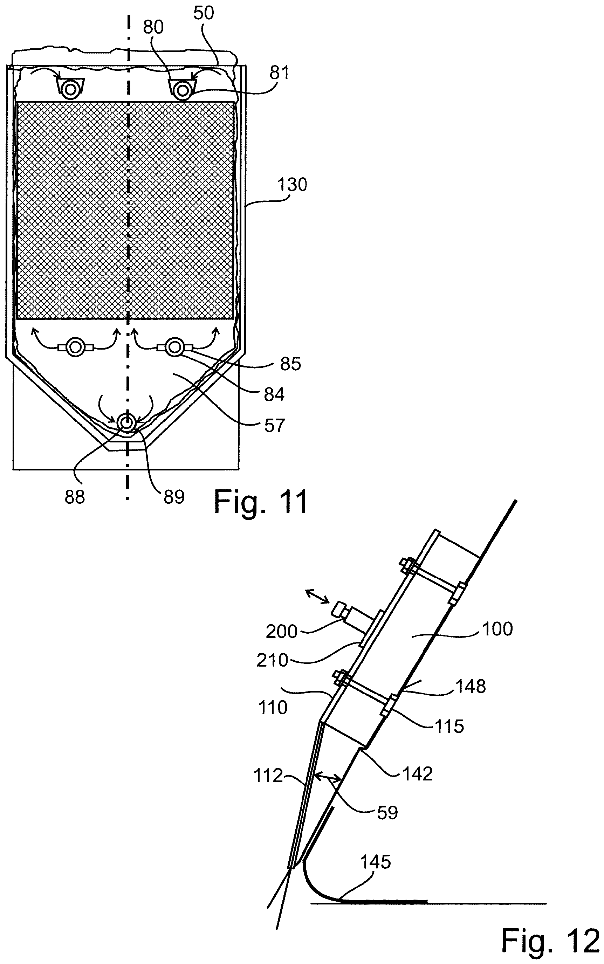

Um den Winkel $\beta$ während des Betriebs zu gewährleisten, wird der erfindungsgemäße Feststoffabscheider für den Betrieb auf einem Gestell 140 befestigt (Fig. 11 bis 13).

Üblicherweise umfasst das Gestell 140 einen Gestellfuß 145 und eine Auflage 148 mit einem vordefinierten Winkel 10 (= $\beta$) zur Stellfläche. Auf der Auflage 148 wird das Lamellenpaket 1

inkl. Stützplatte 30 mit Hilfe eines Vorsprungs 142 und / oder Deckels 110 sowie Befestigungselementen 115 auf einer vordefinierte Höhe festgehalten, damit sowohl der Erntestromsammelbereich 56 (oben) und der Feststoffsammelbereich 57 während des Betriebs möglichst faltenlos auf der Auflage ruhen können. Hiermit werden tote Räume und entsprechendes Fouling vermindert.

In einer bevorzugten Ausführungsform verfügt das Gestell 140 über ein Gehäuse 100 und einen Deckel 110 zur Aufnahme des Lamellenpakets 1.

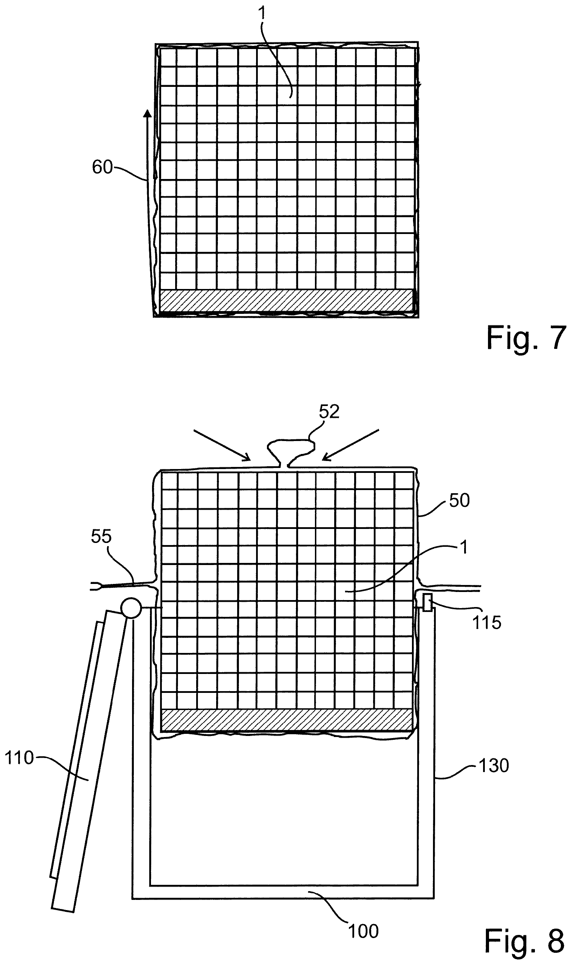

In diesem Fall kann der Straffungsprozess auch beim Einbau des erfindungsgemäßen Feststoffabscheiders auf das Gestell 140 und insbesondere in dem Gehäuse 100 und Deckel 110 (Fig. 6 und 7) ggf. auch ohne Umwicklung mit einem Befestigungsband 60 stattfinden. Hierbei wird der Kunststoffbeutel 50 mit Hilfe des Gehäuses 100 an die Stützplatte 30 und an das Lamellenpaket 1 in Position gehalten und die Falte 52 wird mit Hilfe des Deckels 110 an das Lamellenpaket 1 angepresst. Vorzugweise ist der Deckel 110 an dem Gehäuse 130 an einer Seite z.B. mittels Scharnieren und an der anderen Seite mittels einem oder mehreren verschließbaren Befestigungselementen 115 befestigt. Hiermit ist das Gestell 140 für die Inbetriebnahme des erfindungsgemäßen Feststoffabscheiders einfacher zu betätigen.

In einer bevorzugten Ausführungsform verfügt der Deckel 110 über eine Verlängerung 112 und / oder einen Rahmen 130, die den konisch verjüngten Feststoffsammelbereich 57 in Form, insbesondere den Winkel 59 konstant, hält und dessen Ausdehnen im gefüllten Zustand während des Betriebs verhindern. Ein solcher formpassender Behälter ist u. a. vorteilhaft für den Betrieb des Systems bei größeren hydrostatischen Kräften, wie sie beim Anschluss an große Bioreaktoren zu erwarten sind.

Der erfindungsgemäße Feststoffschrägkanalabscheider **ist** zur **Vermeidung der** Reinigungsproblematik bevorzugt als Einwegartikel ausgeführt.

Die Lagerung der erfindungsgemäßen Feststoffschrägkanalabscheider ist platzsparend, da sie problemlos aufeinander gestapelt werden können und erst bei der Inbetriebnahme mit dem passenden Winkel aufgestellt werden. Sie lassen sich dann einfach außerhalb eines Bioreaktors anschließen und betreiben.

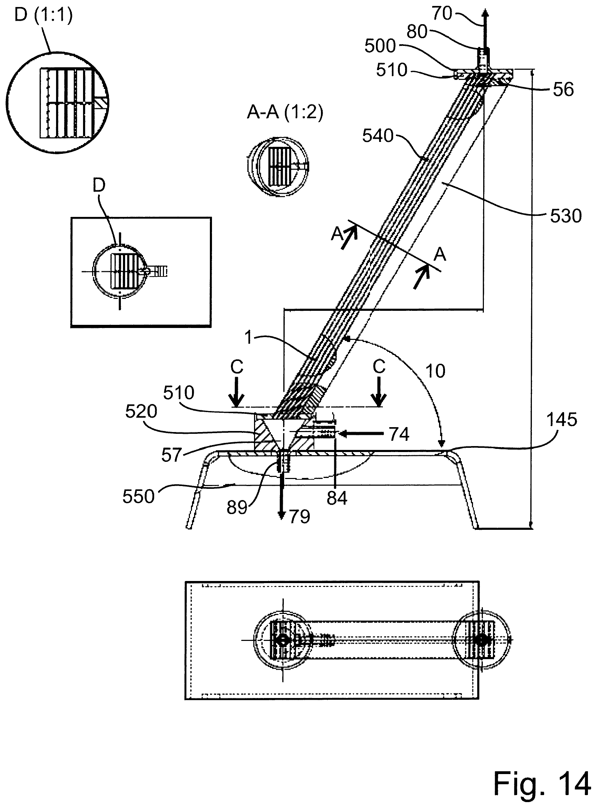

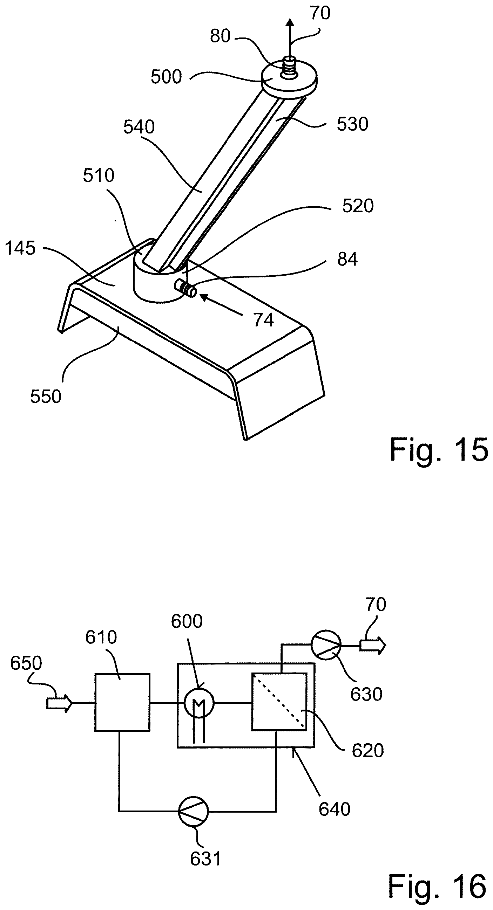

**In einer weiteren Ausführungsform** (Fig. 14 und 15) ist der obere Bereich des erfindungsgemäßen Feststoffschrägkanalabscheiders ein **Sammler,** der beispielsweise aus einem Stück mit Schlauchanschluss durch Zerspanen eines Kunststoffvollstabes insb. aus Polycarbonat wie Makrolon® auf einer Drehmaschine gefertigt wird. Eine weitere Methode, die in erster Linie bei hohen Stückzahlen geeignet ist, ist das Spritzgussverfahren. Der Sammler weist Durchführungen (80), mindestens eine Durchführung zum Abziehen eines von den Zellen getrennten Erntestromes (70) (=Harvest) verbunden mit dem Erntestromsammelbereich 56. Der Erntestromsammelbereich 56 ist durch eine Aussparung in dem Sammler gebildet, die in die Durchführung 80 zum Abziehen eines von den Zellen getrennten Erntestromes (70) mündet. Der Querschnitt dieser Aussparung ist üblicherweise rund oder viereckig. Bevorzugt ist er an die Größe der Öffnung der oberen Steckplatte und hiermit an die Kantendimensionen des Stegplattengrundkörpers angepasst. Die Höhe der Aussparung wird hinsichtlich der Minimierung des Totvolumens und Optimierung der Strömungsführung angepasst. Sie beträgt üblicherweise 1 bis 5 mm. Diese Aussparung kann auch trichterförmig sein.

In dieser Ausführungsform werden das obere und das untere Ende des Stegplattengrundkörpers in sog. Steckplatten eingeführt und verklebt. Auch die Steckplatten werden üblicherweise durch Zerspanen auf einer Dreh- und Fräsmaschine oder im Spritzgussverfahren gefertigt. Sie sind vorzugsweise aus dem Material des Sammlers. Sie weisen eine viereckige Öffnung vorzugsweise mit einem vordefinierten Winkel zum Einstecken der unteren und oberen Enden des Stegplattengrundkörpers. Sie sind üblicherweise rund.

Vorzugsweise bildet ein **Trichter** das untere Segment und den Feststoffsammelbereich (57), der eine oder mehrere Durchführungen 89 zum Abziehen der Feststoffe aufweist. Der Trichter wird üblicherweise mit einem unteren Schlauchanschluss durch Zerspanen auf einer Drehmaschine oder im Spritzgussverfahren gefertigt. Er ist vorzugsweise aus dem Material der Steckplatten. Der obere Bereich des Trichters weist eine oder mehrere Durchführungen oder Einbauten 84 zur gleichmäßigen Strömungsverteilung der Zellkulturlösung (=Feed) 74 über eine Einleitfläche 510. Der optionale horizontale Verteiler kann durch zwei oder mehrere geometrisch verteilte üblicherweise zwei gegenüberliegende Bohrungen gebildet werden, in den seitliche Anschlüsse eingeklebt werden. Der Trichter wird mit der unteren Steckplatte verklebt.

Bevorzugt weist der nach unten verjüngte, insb. konisch oder pyramidal, Feststoffsammelbereich (57) einen Winkel (58, 59) von 10° bis 60° gegen die Vertikale auf. Die Winkel 58 und 59 können separat ausgewählt werden.

Für die mechanische Stabilität des Feststoffschrägkanalabscheiders sind üblicherweise der Sammler, die Steckplatten und der Trichter biegefest.

Vorzugsweise wird der Stegplattengrundkörper mittels einer **Versteifungslasche** stabilisiert. Die **Versteifungslasche** wird durch Verklebung mit dem Stegplattengrundkörper sowie an der oberen und unteren Steckplatte angebracht und gewährleistet eine ausreichende mechanische Stabilität des Feststoffschrägkanalabscheiders..

Im Vergleich zu den Edelstahl Schrägkanalabscheidern aus dem Stand der Technik entfällt bei den erfindungsgemäßen Feststoffschrägkanalabscheider (=Kunststoffplattenabscheider) die aufwändige Fertigung des Grundkörpers (spanende Bearbeitung, zahlreiche Schweißschritte sowie das spiegelglatt Elektropolieren). Das aufwändige Schweißen wird durch einfaches Kleben ersetzt. Die Herstellung des erfindungsgemäßen Schrägkanalabscheiders erfordert keine Verschraubung und keine Dichtung. Insgesamt werden bei der Herstellung des Kunststoffplattenabscheiders im Vergleich zum Edelstahl Schrägkanalabscheider erhebliche Zeit-und Materialkostenvorteile erreicht.

Dieser erfindungsgemäße Feststoffschrägkanalabscheider wird üblicherweise folgendermaßen hergestellt:

    a. Zuschneiden insbesondere Sägen des Stegplattengrundkörpers,
    b. Entgraten und Reinigen des Stegplattengrundkörpers,
    c. Herstellen folgender Komponenten:

        1) Trichter
        2) Zwei Steckplatten
        3) Sammler
        4) Vorzugsweise eine Versteifungslasche

    d. beidseitiges Einführen des Stegplattengrundkörpers in die Steckplatten, und Verkleben vorzugsweise mit einer UV-härtenden Klebstoff wie z. B. Loctite 3211,
    e. Verkleben der oberen Steckplatte mit dem Sammler,
    f. Verkleben der unteren Steckplatte mit dem Trichter,
    g. Anbringen und Verkleben der Versteifungslasche.

Für den Betrieb erfolgt üblicherweise eine Befestigung des Feststoffschrägkanalabscheiders auf einer Konsole.
Bedingt durch den Kunststoffbau und das dadurch reduzierte Gewicht reicht je nach Größe zum Aufstellen eine wiederverwendbare Konsole aus Edelstahl. Für einen Schrägkanalabscheider üblicher Größe (Abscheiderfläche von 0,15 m$^2$) wurde ein gesamtes Gewicht inkl. Konsole von ca. 4 kg erreicht (Vergleich Edelstahl gleicher Größe circa 40 kg). Hiermit ist der erfindungsgemäße Einweg-Schrägkanalabscheider leicht transportierbar und erfordert kein fahrbares Transportgestell.

Üblicherweise werden die erfindungsgemäßen Feststoffschrägkanalabscheider an einem Bioreaktor z. B. an einem Einweg Bioreaktor wie in US 2009-0180933 beschrieben extern mittels Schlauchleitungen angekoppelt. Die Versorgung des erfindungsgemäßen Abscheiders wird durch mindestens zwei Pumpen, bevorzugt scherarmen Schlauchpumpen, sichergestellt (Fig. 18). Die Pumpen ermöglichen die Entnahme der Zellkulturlösung aus dem Bioreaktorraum, deren Zuleitung nach Kühlung über einen Wärmeaustauscher zur Abscheidevorrichtung, die Entnahme des Erntestroms aus der Abscheidevorrichtung und den Rücktransport des Feststoffstroms (=Retum 70) zum Bioreaktor. Die erforderlichen Abscheideflächen richten sich nach den Sedimentationseigenschaften der Zellen sowie den angestrebten Perfusionsraten und Zellkonzentrationen. Bevorzugte Perfusionsraten liegen im Bereich von 0,1 bis 40 1/Tag, besonders bevorzugte von 0,5 bis 20 1/Tag. Bevorzugte Abscheideflächen pro Bioreaktorvolumen liegen je nach Sedimentationseigenschaften der Zellen (abhängig von der Konzentration, Größe und Agglomerationsneigung der Zellen) im Bereich von 0,1 bis 100 m$^2$/ m$^3$ besonders bevorzugte von 2 bis 20 m$^2$/m$^3$.

Die beschriebenen Verfahren erlauben die einfache und kostengünstige Herstellung des erfindungsgemäßen Feststoffschrägkanalabscheiders zur Rückhaltung und Rückführung von Zellen. Durch die in weiten Grenzen variierbare Konfiguration der Lamellenpakete lässt sich die Geometrie der späteren Vorrichtung einfach und genau festlegen und im Gegensatz zu Systemen aus Edelstahl auch für sehr große Bioreaktoren bereitstellen. Die beschriebenen Verfahren erlauben insbesondere die kostengünstige Herstellung von Einwegelementen, durch deren Einsatz der Aufwand für die Bereitstellung eines nach den Pharmagrundsätzen gereinigten Rückhaltesystems auf ein Minimum reduziert werden kann.

Der Anschluss an die Fermenter erfolgt mittels am Ende der Schlauchleitungen befestigter Steril-Kuppler diverser Hersteller (Fa. Pall, Fa. Sartorius, Fa. Coulder) innerhalb oder außerhalb von Sicherheitswerkbänken, vorzugsweise jedoch durch Schlauchschweißen. **Die** an den erfindungsgemäßen Feststoffabscheidern befestigten Schlauchleitungen sind daher bevorzugt - zumindest teilweise - mit einem das Schlauchschweißen geeigneten Schlauchelement ausgestattet. Außerdem enthalten üblicherweise die Schlauchleitungen zur Förderung der Suspension mindestens zwei spezielle mechanisch hoch belastbare Schlauchelemente (z.B. aus Elastomerschlauch Verderprene der Fa. Verder), die nicht-invasiv in Schlauchpumpen eingelegt werden können, ohne die Sterilität der Abscheider zu gefährden. Der Anschluss, der Betrieb und die Wartung sind problemlos. Die Ausführung der erfindungsgemäßen Vorrichtung oder Teile der erfindungsgemäßen Vorrichtung als Einwegelement eliminiert Reinigungsprobleme.

Zur Verbesserung des Abrutschverhaltens der Zellen in den Kanälen des Lamellenpakets und auf den Innenwänden des konisch verjüngten Feststoffsammelbereichs kann die Vorrichtung mit geeigneten Mitteln, beispielsweise pneumatischen oder elektrischen Vibratoren, zur Vibration gebracht werden.

Ein direkter Einsatz des Lamellenpakets 1 in aerobe Bioreaktoren ist prinzipiell denkbar, wenn die zur Begasung erforderlichen Gasblasen von den Zutrittsöffnungen ferngehalten werden können. Bevorzugt ist der erfindungsgemäßen Abscheider jedoch für die Verwendung außerhalb eines Bioreaktors vorgesehen.

Weiterer Gegenstand der vorliegenden Erfindung ist eine Bioreaktoranlage bestehend aus einem Bioreaktor und einer der beschriebenen erfindungsgemäßen Feststoffschrägkanalabscheider gemäß Anspruch 1.

Bevorzugt ist der Bioreaktor ein Einweg-Reaktor, insbesondere ein wie in US 2009-0180933 beschriebener Reaktor.

Die Bioreaktoranlage ist beispielsweise ein Perfusionsreaktor, der in bekannter Weise betrieben werden kann. Nährmedium wird in den Bioreaktor kontinuierlich zugeführt, und zellarmer Zellkulturüberstand wird kontinuierlich abgeführt. Der Perfusionsreaktor kann bei hohen Perfusionsraten $q/V$ (Mediendurchsatz $q$ pro Bioreaktorvolumen $V$) betrieben werden, wenn dies biologisch sinnvoll ist und eine ausreichende Abscheidefläche zur Verfügung gestellt wird. In diesem Fall erfolgt die Durchströmung des Abscheiders kontinuierlich.

Ebenso kann der Perfusionsreaktor derart betrieben werden, dass man eine Kultur zunächst absatzweise hochwachsen lässt. Wenn das Medium so weit verbraucht ist, dass kein nennenswerter Aufbau von Biomasse mehr möglich ist, zieht man über den externen Zellenabscheider Kulturüberstand ab, der nahezu frei von Biomasse ist. Den im Bioreaktor gewonnen Raum kann man dann nutzen, um frisches Nährmedium zuzuführen, wodurch weiteres Wachstum und damit eine höhere Gesamtbiomasseproduktivität ermöglicht werden (Repeated-Batch-Modus). In diesem Fall erfolgt die Durchströmung des Zellenabscheiders absatzweise. Dieses Verfahren bietet sich zum Beispiel für Vorkulturen an, mit denen sehr große Bioreaktoren geimpft werden sollen, da es die Produktivität bestehender Vorkulturreaktoren erhöhen kann.

Für den Betrieb an Bioreaktoren wird eine kontinuierliche Strömung des erfindungsgemäßen Feststoffschrägkanalabscheiders bevorzugt.

Der Bioreaktor oder Perfusionsreaktor kann zur Züchtung von Zellen eingesetzt werden, die in vitro und in freier Suspension oder auf Mikroträger wachsen. Zu den bevorzugten Zellen, gehören Protozoen sowie adhäsive und nicht-adhäsive Eukaryoten Zellen menschlichen (Nerven-, Blut- oder Gewebezellen, sowie Stammzellen embryonaler oder adulter Herkunft), tierischen oder pflanzlichen Ursprungs, die z.B. durch gentechnische Veränderung befähigt sind, spezielle pharmazeutische Wirkstoffe wie Viren, Proteine, Enzyme, Antikörper, Neuronen, Gewebezellen oder diagnostische Strukturen zu produzieren. Besonders bevorzugt werden für die pharmazeutische Hochleistungsproduktion geeignete Zellen verwendet, zum Beispiel Ciliaten, Insektenzellen, Baby Hamster Kidney (BHK) Zellen, Chinese Hamster Ovary (CHO) Zellen, HKB-Zellen (durch die Fusion von humaner HEK 293-Zelllinie mit der humanen Burkitt Lyphomzelllinie 2B8 entstanden), Hybridoma Zellen sowie Stammzellen.

In einer alternativen Ausführungsform der Anlage ist einer der beschriebenen erfindungsgemäßen Zellenabscheider im absatzweise durchgeführten Betrieb nach Beendigung der Fermentation vor der Zellabtrennung an einen weiteren Bioreaktor oder einen Erntetank angeschlossen mit dem Ziel, die auf die Filter aufzutragende Zellmasse und damit die erforderlichen Filterflächen zu reduzieren.

Das Verfahren zur Rückhaltung und Rückführung von Feststoffen, insbesondere Zellen, erfolgt in dem durchströmten Feststoffschrägkanalabscheider, wobei dem Feststoffschrägkanalabscheider kontinuierlich oder absatzweise feststoffenthaltendes Medium zugeführt und feststofffreies Medium abgeführt wird, wobei eine Strömungsgeschwindigkeit herrscht, welche die Wahrung laminarer Strömungszustände gemäß Re < 2300 gestattet, womit eine effizienzmindernde Resuspendierung der abgeschiedenen Zellen gegen das Erdschwerefeld vermieden wird.

Die Reynolds-Zahl Re kann nach Gl. 7 aus der über den Querschnitt gemittelten Strömungsgeschwindigkeit $w$, der kinematischen Viskosität $\nu$ des strömenden Mediums und dem Innendurchmesser $d$ eines Kanals berechnet werden:

$$\mathrm{Re} = (w \cdot d / \nu) \qquad \text{(Gl. 7)}$$

In schrägen Kanälen herrscht an den Kanalinnenwänden eine geringere Strömungsgeschwindigkeit als in den Kanalmitten. Die Zellen sedimentieren in den Kanälen und rutschen an der Unterseite der Kanäle entgegen der Strömungsrichtung den unteren Kanalenden entgegen. Die von den Zellen befreite Zellkulturlösung wird durch die Kanäle in einen Erntestromsammelbereich 56 abgegeben, der oberhalb der Kanäle angeordnet ist, und schließlich aus dem Gefäß befördert.

Das erfindungsgemäße Verfahren kann vorzugsweise außerhalb eines Bioreaktors ausgeführt werden. Hierzu wird die Zellkulturlösung mit Zellen aus dem Bioreaktor in den erfindungsgemäßen Zellenabscheider befördert. Bevorzugt werden die Zellen vor Eintritt in den Abscheider in einem externen Gefäß gekühlt, um den Stoffwechsel zu verlangsamen und somit einer produktivitätsmindernden Unterversorgung der Zellen entgegenzuwirken. In gekühlter Suspension ist eine Sauerstoffversorgung der sedimentierenden Zellen nicht erforderlich. Zumeist ist eine Abkühlung der Zellkulturlösung auf die Umgebungstemperatur der Abscheider völlig ausreichend, so dass neben dem gewünschten Stoffwechseleffekt Konvektionsströmungen sicher vermieden werden. Zur Überwachung der ausreichenden Versorgung der Zellen kann der Abscheider mit mindestens einem Einwegsensor z:B. zur Messung der Sauerstoffkonzentration und/oder des pH-Wertes ausgestattet werden. Eine Unterbringung der Sensoren ist sowohl in den Wänden wie auch den Verbindungsleitung zum Bioreaktor oder den Erntegefäßen möglich.

Bei der Rückhaltung und Rückführung wirken auf die Zellen nur moderate Scherkräfte, die zumeist von den Zellen gut vertragen werden. Die Zellen werden in der Abscheidevorrichtung auf Fermentationstemperatur oder einem erniedrigten Temperaturniveau gehalten und die Versorgung mit Nährstoffen ist gegeben.

Im Folgenden werden Ausführungsbeispiele der Erfindung anhand von Zeichnungen näher erläutert ohne die Erfindung hierauf zu beschränken.

Fig 1. Schematische Darstellung der erfindungsgemäßen Einweg-Feststoffabscheider mit Lamellenpaket.

Fig. 2 Schematische Darstellung eines Lamellenpakets 1 (Längsschnitt)

Fig. 3 Schematische Darstellung eines Lamellenpakets 1 (Längsschnitt)

Fig. 4 Schema des Aufbaus verschiedener Lamellenpakete (Querschnitt AA' von Fig. 3)

Fig. 5 Schema des Aufbringens des Kunststoffbeutels 50 auf einem Lamellenpaket 1 (Querschnitt AA' von Fig. 3)

Fig. 6 und Fig. 7 Straffung und Befestigung des Kunststoffbeutels 50 auf einem Lamellenpaket 1 (Querschnitt)

Fig. 8 und Fig. 9 Alternative Straffung und Befestigung des Kunststoffbeutels 50 auf einem Lamellenpaket 1 mit Hilfe von Rahmen 130 und Deckel 110 (Querschnitt)

Fig. 10 Seitenansichten der erfindungsgemäßen Feststoffabscheider mit Lamellenpaket 1 auf Gestell 140.

Fig. 11 Frontansichten der erfindungsgemäßen Feststoffabscheider mit Lamellenpaket 1 auf Gestell 140.

Fig. 12 Längsschnitte der erfindungsgemäßen Feststoffabscheider mit Lamellenpaket 1 auf Gestell 140 mit Rahmen 130 und Deckel 110.

Fig. 13 Frontansichten der erfindungsgemäßen Feststoffabscheider mit Lamellenpaket 1 auf ihrem Gestell 140 mit Rahmen 130 und Deckel 110.

Fig 14. Längsschnitt des erfindungsgemäßen Feststoffabscheider auf seiner Konsole, Sicht von oben, Querschnitte (A-A, C-C) und Vergrößerungen (D).

Fig 15. Schematische 3-dimensionale Darstellung der erfindungsgemäßen Feststoffabscheider auf seiner Konsole

Fig. 16 Verfahrensschema eines Perfusionsreaktors. Um die respiratorische Aktivität der Zellen im Bioreaktorablauf zu reduzieren, wird dessen Temperatur möglichst unmittelbar nach dem Abzug in einer Kühlvorrichtung auf ein niedrigeres Niveau abgesenkt. Auf diese Weise wird verhindert, dass die Zellen in der Zellenabscheider zu lange in einem Sauerstoff-limitierten Zustand verweilen, was die Zellen physiologisch beschädigen könnte. Im gezeigten Bespiel besteht der Abscheider 640 aus einem Abscheidebeutel 620 und einer integrierten Kühlvorrichtung 600. Die Flüssigkeitsströme zwischen Bioreaktor 610 und Abscheider 640 werden durch die scherarmen Pumpen 630 und 631 eingestellt.

Andere Verschaltungen, z.B. die Positionierung von einer der beiden Pumpen 630 und 631 im Bioreaktorablauf, sind ebenso denkbar.

**Bezugszeichen:**

| 1 | Lamellenpaket / Abscheiderfläche |
|---|---|
| 5 | Stegbreite |
| 8 | Plattenabstand |
| 10 | Winkel |
| 13 | Länge |
| 15 | Breite |
| 18 | Höhe |
| 30 | Stützplatte |
| 50 | Kunststoffbeutel |
| 52 | Überschuss / Falte |
| 55 | Schweißnaht |
| 56 | Erntestromsammelbereich |
| 57 | Feststoffsammelbereich |
| 58 | Winkel |
| 59 | Winkel |
| 60 | Befestigungsband |
| 70 | Erntestrom (Harvest) |
| 74 | Bioreaktorgemisch / Feed |
| 79 | Rückführung |
| 80 | Durchführung |
| 81 | Strömungsinverter |
| 84 | Durchführung |
| 85 | Horizontalverteiler |
| 86 | Einlassströmung |
| 88 | zentrale Absaugung |
| 89 | Durchführung |
| 90 | Auschlussplatte |
| 100 | Gehäuse |
| 110 | Deckel |
| 112 | Verlängerung |
| 115 | Befestigungselement |
| 130 | Rahmen |
| 140 | Gestell |
| 142 | Vorsprung |
| 145 | Gestellfuß |
| 148 | Auflage |
| 200 | Vibrator |
| 210 | Montageplatte |

EP 2 817 405 B1

Profile eines Lamellenpakets

| | |
|---|---|
| 311 | Lamellenpaket |
| 320 | Rechteckprofil |
| 321 | Lamellenpaket |
| 330 | Rundprofil |
| 331 | Lamellenpaket |
| 340 | Rundprofil |
| 341 | Lamellenpaket |
| 350 | 6-Eck-Profil |
| 351 | Lamellenpaket |

| | |
|---|---|
| 500 | Sammler |
| 510 | Steckplatten |
| 520 | Trichter |
| 530 | Versteifungslasche |
| 540 | Stegplattengrundkörper |

| | |
|---|---|
| 550 | Konsole |
| 600 | Kühlvorrichtung |
| 610 | Bioreaktor |
| 620 | Abscheidevorrichtung |
| 630, 631 | Pumpen |
| 640 | Abscheider = Abscheidebeutel + Kühlvorrichtung ggf. im Gestell oder Behälter integriert. |
| 650 | Kulturmedium |

**Patentansprüche**

1. Feststoffschrägkanalabscheider zur Rückhaltung und Rückführung von Feststoffen aus einem Reaktorgemisch, umfassend folgende Elemente:

- einen oberen Bereich des Feststoffabscheiders mit einer oder mehreren Durchführungen/Einbauten (80) zum Abziehen eines von den Zellen getrennten Erntestromes (70) (=Harvest) aus einem Erntestromsammelbereich (56), verbunden mit
- einem Abscheidebereich gebildet durch ein Lamellenpaket (1) aus ein oder mehrlagigen Kunststoffstegplatten, das während des Betriebs mit einem Winkel (10) von 30° bis 80° zur Horizontalen geneigt ist, verbunden mit
- einem unteren Segment des Feststoffabscheiders mit einer oder mehreren Durchführungen oder Einbauten (84) zur Strömungsverteilung des Reaktorgemisches (74), über
- einen nach unten verjüngten Feststoffsammelbereich (57) zum Sammeln der Zellen mit Hilfe der Schwerkraft,

wobei der Feststoffsammelbereich (57) konisch oder pyramidal nach unten verjüngt ist.

2. Feststoffschrägkanalabscheider nach Anspruch 1, wobei der Feststoffsammelbereich (57) eine oder mehrere Durchführung (89) oder Einbauten (88) zum Abziehen der Feststoffe aufweist.

3. Feststoffschrägkanalabscheider nach Anspruch 1 bis 2, der mindestens ein Einwegsensor im Innenraum umfasst.

4. Feststoffschrägkanalabscheider nach einem der Ansprüche 1 bis 3, wobei der Abscheidebereich aus einer Vielzahl von nebeneinander angeordneten Kanälen in dem Lamellenpaket (1) besteht.

5. Feststoffschrägkanalabscheider nach einem der Ansprüche 1 bis 4, wobei das Verhältnis von Steghöhe zur Kanalbreite hs/d $0{,}01 \leq hs/d \leq 5$ beträgt, mit der Einschränkung, dass beide Abmessungen hs und d jeweils größer als oder gleich 3 mm sind.

6. Feststoffschrägkanalabscheider nach einem der Ansprüche 1 bis 5 umfassend einen durchströmten gamma-sterilisierbaren Kunststoffbeutel (50) und innerhalb des Kunststoffbeutels (50):

- im oberen Bereich des Kunststoffbeutels (50) die Durchführungen/Einbauten (80) zum Abziehen eines von den Feststoffen getrennten Erntestroms (70) aus dem Erntestromsammelbereich (56),
- im oberen Segment eines mittleren Bereiches des Kunststoffbeutels (50) der Abscheidebereich mit Lamellenpaket (1) aus ein oder mehrlagigen Kunststoffstegplatten,
- im unteren Segment des mittleren Bereiches des Kunststoffbeutels (50) die Durchführungen oder Einbauten (84) mit horizontalen Verteiler (85) zur gleichmäßigen horizontalen Strömungsverteilung der Zellkulturlösung (74) über eine Einleitfläche (510),
- im unteren Bereich des Kunststoffbeutels (50) den nach unten konisch verjüngten Feststoffsammelbereich (57) zum Sammeln der Feststoffe mit Hilfe der Schwerkraft.

7. Feststoffschrägkanalabscheider nach Anspruch 6, wobei die Kanäle eine Kanallänge L von 30 % bis 95% einer Länge LK des Kunststoffbeutels aufweisen.

8. Feststoffschrägkanalabscheider nach einem der Ansprüche 6 oder 7, umfassend einen Behälter zur Aufnahme des Feststoffabscheiders, wobei der Behälter mindestens einen Innenraum zur Aufnahme des Feststoffabscheiders aufweist, wobei dieser Innenraum an die Form der Feststoffabscheiders mittels an die Form des Feststoffabscheider angepasste Wände umfasst, die Wände den Innenraum einschließen und von der Außenwelt abgrenzen, eine Öffnung zur Einführung des Feststoffabscheiders von oben in den Behälter, umfasst.

9. Feststoffschrägkanalabscheider nach einem der Ansprüche 1 bis 5, wobei:

- der oberen Bereich des Feststoffabscheiders ein Sammler mit einem Erntestromsammelbereich (56) ist, verbunden mit
- einen Abscheidebereich gebildet durch ein Lamellenpaket (1) aus ein oder mehrlagigen Kunststoffstegplatten einen Stegplattengrundkörper bildet, der, oben und unten eingesteckt in Steckplatten ist, und verbunden ist mit
- einem unteren Segment des Feststoffabscheiders mit einer oder mehreren Durchführungen oder Einbauten (84) zur Strömungsverteilung des Reaktorgemisches (74), über
- einen unten konisch verjüngten Feststoffsammelbereich (57) zum Sammeln der Zellen mit Hilfe der Schwerkraft,
- wobei der untere Segment und der unten konisch verjüngten Feststoffsammelbereich (57) ein Trichter ist, und
- wobei alle Elemente des Feststoffabscheiders aus Kunststoff sind.

10. Bioreaktoranlage umfassend einen Bioreaktor verbunden mit einem Feststoffschrägkanalabscheider nach einem der Ansprüche 1 bis 9.

**Claims**

1. Inclined channel solids separator for retaining and recirculating solids from a reactor mixture, comprising the following elements:

- an upper region of the solids separator having one or more feed-throughs/fittings (80) for removing a harvest stream (70) separated from the cells (=harvest) from a harvest stream collection region (56), connected to
- a separation region formed by a plate stack (1) composed of single-layer or multilayer plastic web plates, which stack is tilted during operation at an angle (10) of from 30° to 80° with respect to the horizontal, connected to
- a lower segment of the solids separator having one or more feed-throughs or fittings (84) for flow distribution of the reactor mixture (74), above
- a solids collection region (57) which is downwardly tapered for collecting the cells by means of gravity,

wherein the solids collection region (57) is downwardly tapered in a conical or pyramidical manner.

2. Inclined channel solids separator according to Claim 1, wherein the solids collection region (57) has one or more feed-throughs (89) or fittings (88) for removing the solids.

3. Inclined channel solids separator according to Claim 1 or 2, which comprises at least one disposable sensor in the interior.

4. Inclined channel solids separator according to any of Claims 1 to 3, wherein the separation region consists of a multiplicity of channels arranged next to one another in the plate stack (1).

5. Inclined channel solids separator according to any of Claims 1 to 4, wherein the ratio of strut height to channel width hs/d is $0.01 \leq hs/d \leq 5$, with the restriction that the two dimensions hs and d are both greater than or equal to 3 mm.

6. Inclined channel solids separator according to any of Claims 1 to 5 comprising a gamma-sterilizable plastic bag (50) through which flow passes and, within the plastic bag (50):

   - in the upper region of the plastic bag (50), the feed-throughs/fittings (80) for removing a harvest stream (70) separated from the solids from the harvest stream collection region (56),
   - in the upper segment of a central region of the plastic bag (50), the separation region with plate stack (1) composed of single-layer or multilayer plastic web plates,
   - in the lower segment of the central region of the plastic bag (50), the feed-throughs or fittings (84) having horizontal distributors (85) for uniform horizontal flow distribution of the cell culture solution (74) via an infeed surface area (510),
   - in the lower region of the plastic bag (50), the solids collection region (57) which is downwardly tapered in a conical manner for collecting the solids by means of gravity.

7. Inclined channel solids separator according to Claim 6, wherein the channels have a channel length L of from 30% to 95% of a length LK of the plastic bag.

8. Inclined channel solids separator according to either of Claims 6 and 7, comprising a container for accommodating the solids separator, wherein the container has at least one interior for accommodating the solids separator, wherein said interior comprises walls matched to the shape of the solids separator by means of to the shape of the solids separator, the walls enclose the interior and demarcate it from the outside world, an opening for introducing the solids separator from above into the container.

9. Inclined channel solids separator according to any of Claims 1 to 5, wherein:

   - the upper region of the solids separator is a collector having a harvest stream collection region (56), connected to
   - a separation region formed by a plate stack (1) composed of single-layer or multilayer plastic web plates forms a web-plate base body, which is plugged into plug plates at the top and bottom and is connected to
   - a lower segment of the solids separator having one or more feed-throughs or fittings (84) for flow distribution of the reactor mixture (74), above
   - a solids collection region (57) which is downwardly tapered in a conical manner for collecting the cells by means of gravity,
   - wherein the lower segment and the solids collection region (57) which is downwardly tapered in a conical manner is a funnel, and
   - wherein all the elements of the solids separator are composed of plastic.

10. Bioreactor unit comprising a bioreactor connected to an inclined channel solids separator according to any of Claims 1 to 9.

**Revendications**

1. Séparateur de solides à canal incliné pour la rétention et le retour de solides à partir d'un mélange de réacteur, comprenant les éléments suivants :

   - une zone supérieure du séparateur de solides comprenant un ou plusieurs passages/composants intérieurs (80) pour le soutirage d'un courant de récolte (70) (= Harvest) séparé des cellules à partir d'une zone de collecte du courant de récolte (56), reliée avec
   - une zone de séparation formée par un paquet de lamelles (1) constitué de plaques alvéolaires plastiques mono- ou multicouches, qui est inclinée à un angle (10) de 30° à 80° par rapport à l'horizontale pendant l'exploitation, reliée avec
   - un segment inférieur du séparateur de solides comprenant un ou plusieurs passages ou composants intérieurs (84) pour la répartition de l'écoulement du mélange de réacteur (74) sur

- une zone de collecte de solides rétrécie vers le bas (57) pour la collecte des cellules à l'aide de la force de pesanteur,

la zone de collecte de solides (57) étant rétrécie vers le bas sous forme conique ou pyramidale.

2. Séparateur de solides à canal incliné selon la revendication 1, dans lequel la zone de collecte de solides (57) comprend un ou plusieurs passages (89) ou composants intérieurs (88) pour le soutirage des solides.

3. Séparateur de solides à canal incliné selon la revendication 1 ou 2, qui comprend au moins un capteur à une voie dans l'espace intérieur.

4. Séparateur de solides à canal incliné selon l'une quelconque des revendications 1 à 3, dans lequel la zone de séparation est constituée d'une pluralité de canaux adjacents les uns aux autres dans le paquet de lamelles (1).

5. Séparateur de solides à canal incliné selon l'une quelconque des revendications 1 à 4, dans lequel le rapport entre la hauteur des traverses et la largeur des canaux hs/d est de $0,01 \leq hs/d \leq 5$, à condition que les deux dimensions hs et d soient chacune supérieures ou égales à 3 mm.

6. Séparateur de solides à canal incliné selon l'une quelconque des revendications 1 à 5, comprenant une poche en plastique (50) traversée et stérilisable par rayons gamma, et, dans la poche en plastique (50) :

   - dans la zone supérieure de la poche en plastique (50), les passages/composants intérieurs (80) pour le soutirage d'un courant de récolte séparé des solides (70) à partir de la zone de collecte du courant de récolte (56),
   - dans le segment supérieur d'une zone centrale de la poche en plastique (50), la zone de séparation comprenant un paquet de lamelles (1) constitué de plaques alvéolaires plastiques mono- ou multicouches,
   - dans le segment inférieur de la zone centrale de la poche en plastique (50), les passages ou composants intérieurs (84) comprenant un répartisseur horizontal (85) pour la répartition horizontale uniforme de l'écoulement de la solution de culture cellulaire (74) sur une surface d'entrée (510),
   - dans la zone inférieure de la poche en plastique (50), la zone de collecte de solides rétrécie vers le bas sous forme conique (57) pour la collecte des solides à l'aide de la force de pesanteur.

7. Séparateur de solides à canal incliné selon la revendication 6, dans lequel les canaux ont une longueur de canaux L de 30 % à 95 % d'une longueur LK de la poche en plastique.

8. Séparateur de solides à canal incliné selon l'une quelconque des revendications 6 ou 7, comprenant un contenant pour la réception du séparateur de solides, le contenant comprenant au moins un espace intérieur pour la réception du séparateur de solides, cet espace intérieur comprenant des parois adaptées à la forme du séparateur de solides, les parois entourant l'espace intérieur et le délimitant de l'extérieur, comprenant une ouverture pour l'introduction du séparateur de solides par le haut dans le contenant.

9. Séparateur de solides à canal incliné selon l'une quelconque des revendications 1 à 5, dans lequel :

   - la zone supérieure du séparateur de solides est un collecteur comprenant une zone de collecte du courant de récolte (56), reliée avec
   - une zone de séparation formée par un paquet de lamelles (1) constitué par des plaques alvéolaires plastiques mono- ou multicouches formant un corps de base à plaques alvéolaires, qui est inséré en haut et en bas dans des plaques enfichables, et relié avec
   - un segment inférieur du séparateur de solides, comprenant un ou plusieurs passages ou composants intérieurs (84) pour la répartition du courant du mélange de réacteur (74), sur
   - une zone de collecte de solides rétrécie sous forme conique vers le bas (57) pour la collecte des cellules à l'aide de la force de pesanteur,
   - le segment inférieur et la zone de collecte de solides rétrécie sous forme conique vers le bas (57) étant un entonnoir, et
   - tous les éléments du séparateur de solides étant en plastiques.

10. Unité de bioréacteur comprenant un bioréacteur relié avec un séparateur de solides à canal incliné selon l'une quelconque des revendications 1 à 9.

Fig. 1

Fig. 2

Fig. 3

Fig. 4

Fig. 5

Fig. 6

Fig. 7

Fig. 8

Fig. 9

Fig. 10

Fig. 11

Fig. 12

Fig. 13

D (1:1)

A-A (1:2)

D

500
80
70
510
540
56
530
A
A
1
C      C
10
510
520
74
57
550      89      79      84      145

Fig. 14

Fig. 15

Fig. 16

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 1994026384 A1
- WO 2003020919 A2
- WO 2009152990 A2
- US 6186932 B1
- US 20090180933 A

**In der Beschreibung aufgeführte Nicht-Patentliteratur**

- **HENZLER, H.-J.** *Chemie-Technik,* 1992, vol. 1, 3
- **H.-J. BINDER.** Sedimentation aus Ein- und Mehrkornsuspensionen in schräg stehenden, laminar durchströmten Kreis- und Rechteckrohren. *Dissertation Berlin,* 1980